# EUROPEAN PATENT SPECIFICATION

(11) **EP 0 325 566 B1**
(45) Date of publication and mention of the grant of the patent: **09.06.1993**
(21) Application number: 89830018.1
(22) Date of filing: 20.01.1989
(51) Int. Cl.: A61F 2/38

(54) **Artificial articulation, in particular an artificial knee-joint**
Künstliches Gelenk, insbesondere für ein künstliches Kniegelenk
Articulation artificielle, notamment pour l'articulation artificielle pour le genou

(30) Priority: 22.01.1988 IT 4756588
(43) Date of publication of application: 26.07.1989
(73) Proprietor: SALUS S.R.L., I-00197 Rome (IT); Ugolini, Filippo, I-00192 Rome (IT)
(72) Inventor: Ugolini, Filippo, I-00192 Rome (IT)
(74) Representative: de Simone, Domenico

(56) References cited:
- CH-A- 610 756
- DE-B- 2 114 287
- FR-A- 2 612 767
- GB-A- 1 457 147
- GB-A- 1 509 366

## Description

This invention relates to an artificial articulation. More particularly this invention relates to a knee-joint suitable to replace the natural knee-joint and so realized as to be connected rigidly to the femoral bone and to the tibial bone and to allow an optimal reproduction to be obtained of motions of the natural knee-joint.

As is well known, a correct deambulation in all situations such as for instance climbing an ascent or descending the stairs or reliefs and the like, or merely walking, is permitted by the presence of such a delicate and complex articulation as that of the knee.

When the knee-joint is no longer able to perform its original task because of some different reasons, it is necessary to replace the knee-joint by means of a surgical operation.

Prostheses available and commonly employed at the present time are not always suitable to reproduce the functionality of the natural articulation satisfactorily, in particular because friction occurring in the mechanical device makes the correct deambulation motion toilsome.

Moreover, prostheses available at present almost always require some surgical adaptations of the prostheses themselves.

Indeed, prostheses realized up to now are mounted in situ by realizing the assembling of the hinge during the surgical operation, and the functions of the rods, of the spacers as well as of the hinge itself are integrated with each other, with very few or no possibilities of adjusting some sizes to the particular requirements that could be found during the operation different from the sizes foreseen before the operation, at the time of diagnosis and of designing the operation itself.

In DE-B-2,114,287 an artificial articulation is described, particularly to replace the bone tissue in a knee articulation, made up of two parts connected by a pin articulation having a stop to limit the oscillation zone, one of said parts being anchored to the femoral bone and the other one to the tibial bone.

In Fr-A-2,612,767 it is described an artificial articulation having an articulated coupling between two hinge members having at least one bearing. The coupling is obtained inserting the end of one rod into a cavity obtained in a hinge member.

In the light of the practical difficulties stemming from the employment of prostheses already known, and in order to obviate their drawbacks, the Applicants have devised and realized an artificial knee-joint which is so mechanically and functionally structured as to allow an articulation to be constructed which is equivalent to that obtained with the natural articulation, and whose members are constructed according to a modular way in order to have the possibility of realizing the most suitable prostheses by sizes and types for the actual surgical requirements, said requirements being exactly determinable just during the operation itself.

Moreover, the artificial articulation proposed according to the present patent application is not affected by remarkable frictional forces between the mechanical parts which it is made up of, so that no difficulties arise in the deambulation motions.

It is a further object of the present invention to provide an artificial articulation whose position can be suitably adjusted independently of the way the femoral and tibial bones of the patient have been amputated.

The solution proposed by the Applicants realizes an articulation which is rigidly connected to the femoral and to the tibial bones so as to allow a hinge bending to be obtained of the two parts through an arc of about 120°, possibly with a small negative angle.

These results are obtained through the realization of an artificial articulation which is made up of two hinge members coupled by means of two rolling bearings and integrally fastened, respectively, to the femoral bone and to the tibial bone, some spacing members being or not interposed for restoring the original length of the leg.

Accordingly, it is a specific object of the present invention an artificial articulation, more particularly a knee-joint comprising a first rod insertable into the cavity of the femoral bone; a first hinge member, connected to an end of said first rod that is opposite to its end that is inserted into the bony cavity; a second rod insertable into the bony cavity of the tibial bone; a second hinge member connected to an end of said second rod that is opposite to its end that is inserted into the bony cavity of the tibial bone; an articulated coupling between said two hinge members having at least one bearing, characterized in that said rods are connected to their respective hinge member by means of a dove-tail joint.

In the latter instance, at least one spacing member can be provided between the rod and the hinge member, such spacer being of variable height depending on the requirements at hand, and allowing the articulation to be positioned correctly according to the extent of surgical ablation.

Said spacing members comprise a dove-tail joint to fit into the dove-tail joints of said rod and said hinge member.

Alternatively, some spacing shims can be arranged on each rod instead of said spacing members.

Said mechanical locking means can be made up of a dowel which possibly is threaded and self-locking, or, in the case of members of cylindrical shape, such means can consist of a self-locking threaded collar or of an elastic ring.

Bearings employed for the coupling between the two hinge members are preferably of the self-lubricating rolling type and they are supplied in number of two.

In order to allow the bearings or other mechanical components to be replaced, the coupling between the two hinge members can also be realized so that it is removable and is locked by cover means which are assembled on said bearings and fastened to their positions by pin means or the like.

This invention will be disclosed in the following just for illustrative and not for limitative purposes with particular reference to the figures of the enclosed drawings, wherein:
Figure 1 is a cross-sectional side view of a particular embodiment of the articulation according to the present invention;
Figure 2 is a cross-sectional front view of the articulation of Figure 1;
Figure 3 is a front view of a first embodiment of the fastening system between the rod and the spacer or the hinge of the articulation;
Figure 4 is a front view of a second embodiment of the fastening system between the rod and the spacer or the hinge of the articulation;
Figure 5 is a front view of a third embodiment of the fastening system between the rod and the spacer or the hinge of the articulation; and
Figure 6 is a front view of a fourth embodiment of the fastening system between the rod and the spacer or the hinge of the articulation.

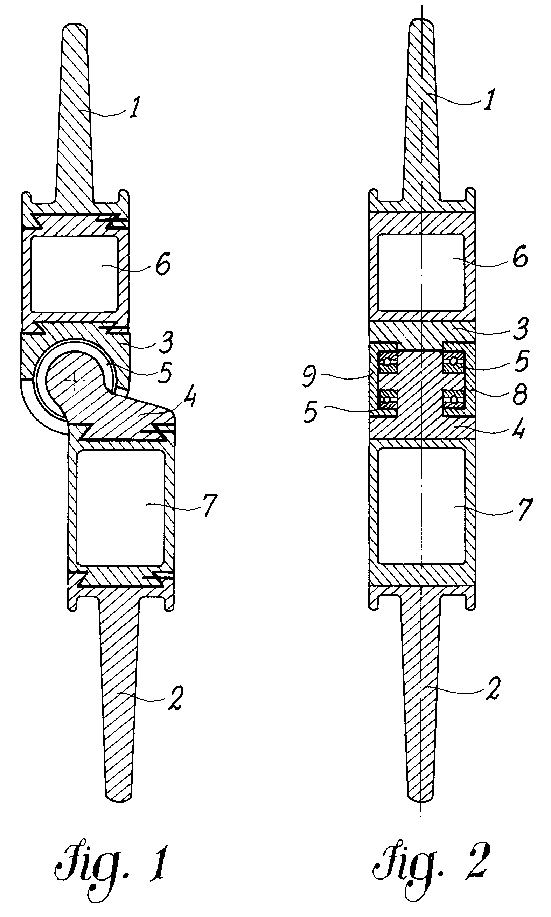

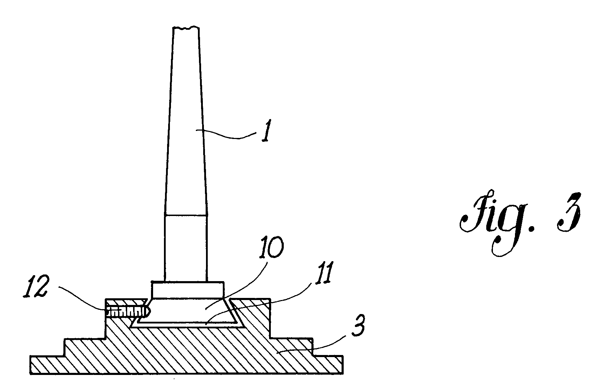

With reference now to Figures 1 and 2, the numeral 1 points out the femoral rod which will be inserted into the central bony cavity of the femoral bone, whereas the numeral 2 points out the tibial rod that will be inserted into the central bony cavity of the tibial bone.

The hinge member which is integral with the rod 1 is pointed out by the reference numeral 3, whereas the rod 2 is integrally coupled to the hinge member 4.

The two hinge members 3 and 4 are so realized as to rotate with respect to one another through the interposition of the rolling bearings 5.

According to the specific cases at hand, the spacers 6 and 7 can be provided between the rod 1 and the hinge member 3, and between the rod 2 and the hinge member 4.

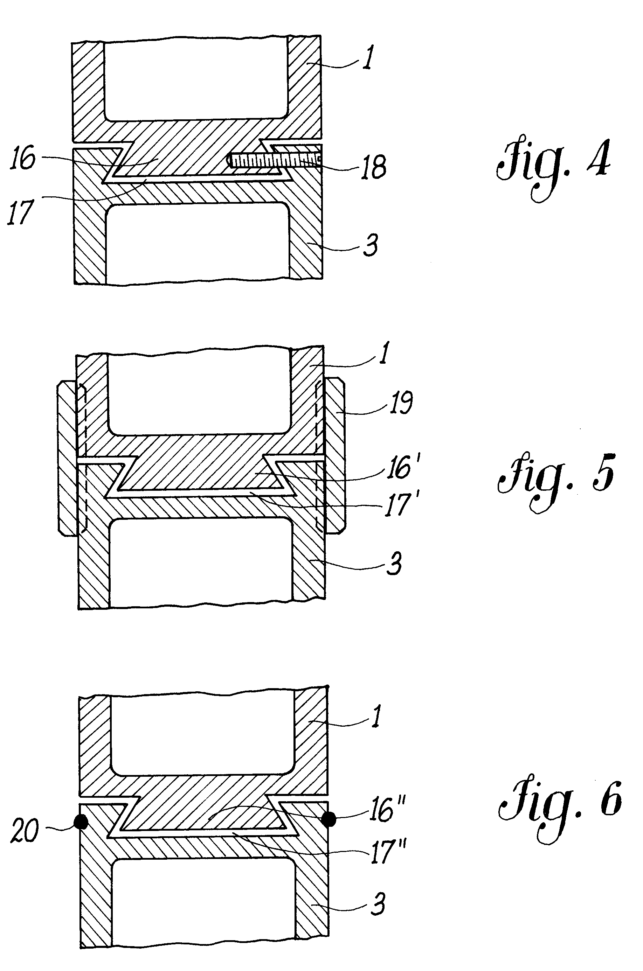

The coupling between the rod 1 or 2 and the spacer 6 or 7 and between the spacer 6 or 7 and the hinge member 3 or 4 is obtained through dovetail joints which will be illustrated more particularly in the following with reference to Figures 3-7.

To allow the surgeon to perform an easier mounting of the prosthesis, the two assemblies consisting of rod 1-(possibly) spacer 6-hinge 3 and rod 2-(possibly) spacer 7-hinge 4 can be mounted separately and then they can be coupled and connected functionally by means of the tow covers 8 and 9 which are kept locked by pins (not shown).

All that also allows the possibility of mounting the articulation to replace one of the rolling bearings or both of them, in case of necessity, or to replace any other component parts.

As can be observed in particular in Figure 1, the axis of the rod 1-spacer 6-hinge 3 assembly and that of the rod 2-spacer 7-hinge 4 assembly are parallel but they are out of alignment, the second axis being shifted forward with respect to the first one, in the direction of the patient's step, so that an absolute stability of the system is obtained when the full body weight is lying on the articulation.

Figures 3-6 show some kinds of embodiment of the coupling between the rod 1 or 2 and the spacer 6 or 7 or the hinge member 3 or 4. Anyway, it is evident that the articulation according to the present invention can also be realized, without the insertion of any spacers, by making the rod 1 and the hing members 3 and the rod 2 and the hinge member 4 integral with each other.

The disclosure of the Figures 3-6 is given with reference to the coupling between the rod 1 or 2 and the hinge members 3 or 4, but it is to be understood that the same coupling can be realized between the spacer 6 or 7 and the rod 1 or 2 or the hinge members 3 or 4.

Figure 3 shows a coupling with a mechanical guide consisting of a dovetail joint 10-11 which is locked by the locking dowel 12.

The dovetail joint (male member 16-female member 17) shown in Figure 4 is locked by a self-locking threaded dowel 18, whereas the similar coupling (members 16' and 17') illustrated in Figure 5 is locked through the employment of a threaded collar 19. Such coupling can only be employed in the case of cylindrical joints.

The elastic ring 20 employed for locking the members 16'' and 17'' of Figure 6 can also be advantageously employed.

Employing such type of solutions to the problem, the spacers 6 and 7 being provided or not, it is possible to replace completely the proper articulation without removing the rods 1 and 2, in the very rare cases when this is necessary.

## Claims

1. An artificial articulation, in particular an artificial knee-joint, comprising a first rod (1) insertable into the cavity of the femoral bone; a first hinge member (3), connected to an end of said first rod (1) that is opposite to its end and that is inserted into the bony cavity; a second rod (2) insertable into the bony cavity of the tibial bone; a second hinge member (4) connected to an end of said second rod (2) that is opposite to its end that is inserted into the bony cavity of the tibial bone; an articulated coupling between said two hinge members (3, 4) having at least one bearing (5), characterized in that said rods (1,2) are connected to their respective hinge member (3,4) by means of a dove-tail joint (10,11; 16,17; 16',17'; 16'',17'').

2. An artificial articulation according to claim 1, characterized in that a spacing member (6,7) is provided between said rod (1,2) and said hinge member (3,4).

3. An artificial articulation according to claims 1 or 2, characterized in that said spacing members comprise a dove-tail joint to fit into the dove-tail joints of said rod (1, 2) and said hinge member (3,4).

4. An artificial articulation according to one of the preceding claims, characterized in that said at least one bearing (5) is a self-lubricating bearing of the rolling-type.

5. An artificial articulation according to claim 1 or 4, characterized in that two bearings (5) are provided.

6. An artificial articulation according to one of the preceding claims, characterized in that a locking means is provided (12; 18; 19; 20) to mechanically locking said coupling.

7. An artificial articulation according to claim 6, characterized in that said locking means includes a dowel (12).

8. An artificial articulation according to claim 7, characterized in that said dowel (12) is of the self-locking threaded type.

9. An artificial articulation according to claim 6, characterized in that said locking means includes a self-locking threaded collar (19).

10. An artificial articulation according to claim 6, characterized in that said locking means includes an elastic ring (20).

11. An artificial articulation according to one of the preceding claims, characterized in that the two hinge members (3,4) are shaped so that the vertical axis of the first rod (1) and the vertical axis of the second rod (2) are parallel, but the second axis is shifted forwards with respect to the first axis in the direction of the patient's step.

12. An artificial articulation according to one of the preceding claims, characterized in that spacing clims are provided on said rods (1,2).

13. An artificial articulation according to one of the preceding claims, characterized in that the locking means include covers (8,9) which are assembled laterally on said bearings and kept fastened by pins.

## Patentansprüche

1. Kuenstliches Gelenk, insbesondere ein kuenstliches Kniegelenk, mit einer ersten Stange (1) zur Einfuehrung in die Hoehle des Oberschenkelsknochen; einem ersten Scharnierglied (3), das mit dem, dem in die Hoehle des Oberschenkelsknochen eingefuehrten Ende entgegengesetzten Ende des vorgenannten ersten Stange (1) verbunden ist; einem zweiten Stange (2), die in die Hoehle des Schleubeines einsetzbar ist; einem zweiten Scharnierglied (4), das mit dem, dem in die Hoehle des Schleubeines eingefuehrten Ende entgegengesetzten Ende des vorgenannten zweiten Stabes (2) verbunden ist; einer Gelenk-Kupplung zwischen den vorgenannten Scharniergliedern (3,4) mit mindestens einem Lager (5), dadurch gekennzeichnet, dass die vorgenannten Stagen (1,2) mit deren entsprechenden Scharniergliedern (3,4) durch eine schwalbenschwanzartige Kupplung (10,11; 16,17; 16',17'; 16'',17'') verbunden sind.

2. Kuenstliches Gelenk nach Anspruch 1, dadurch gekennzeichnet, dass zwischen der vorgenannten Stange (1,2) und dem vorgenannten Scharnierglied (3,4) ein Abstandhalter (6,7) vorgesehen ist.

3. Kuenstliches Gelenk nach Anspruch 1 oder 2, dadurch gekennzeichnet, dass die vorgenannten Abstandhalter (6,7) einen schwalbenschwanzartigen Einschnitt zur Kupplung mit den schwalbenschwanzartigen Einschnitt zur Kupplung mit den schwalbenschwanzartigen Einschnitt der vorgenannten Stangen (1,2) und Scharnierglieder (3,4) aufweisen.

4. Kuenstliches Gelenk nach einem der vorhergehenden Ansprueche, dadurch gekennzeichnet, dass mindstens ein Lager (5) ein selbstschmierendes Waelzlager ist.

5. Kuenstliches Gelenk nach Anspruch 1 oder 4, dadurch gekennzeichnet das zwei Lager (5) vorgesehen sind.

6. Kuenstliches Gelenk nach einem der vorhergehenden Anspruechen, dadurch gekennzeichnet, dass ein Spannmittel (12,18,19,20) zur mechanischen Spannung der vorgenannten Kupplung vorgesehen ist.

7. Kuenstliches Gelenk nach Anspruch 6, dadurch gekennzeichnet, dass das vorgenannte Spannmittel einen Stift (12) enthaelt.

8. Kuenstliches Gelenk nach Anspruch 6, dadurch gekennzeichnet, dass der vorgenannte Stift (12) ein selbstsperrende Haltestift ist.

9. Kuenstliches Gelenk nach Anspruch 6, dadurch gekennzeichnet, dass das vorgenannt Spannmittel einen selbstsperrenden Gewindering (19) enthaelt.

10. Kuenstliches Gelenk nach Anspruch 6, dadurch gekennzeichnet, dass des vorgenannte Spannmittel einen elastischen Ring (20) enthaelt.

11. Kuenstliches Gelenk nach einem der vorhergehenden Ansprueche, dadurch gekennzeichnet, dass die beiden Scharnierglieder (3,4) so ausgebildet sind, dass die senkrechte Achse der ersten Stange (1) und die senkrechte Achse der zweiten Stange (2) zu einander parallel verlaufen, wobei aber die zweit Achse gegenueber der ersten Achse in Richtung des Gehens des Patienten vorgeschoben ist.

12. Kuenstliches Gelenk nach einem der vorhergehenden Anspruechen, dadurch gekennzeichnet, dass auf den vorgenannten Stangen (1,2) Abstandhalter vorgesehen sind.

13. Kuenstliches Gelenk nach einem der vorhergehenden Anspruechen, dadurch gekennzeichnet, dass die Spannmittel seitlich auf den vorgenannten Lagern montierte Deckel aufweisen, die durch Stifte befestigt sind.

## Revendications

1. Articulation artificielle, notamment pour l'articulation artificielle pour le genou, comprenant une premiere barre (1) insérable dans la cavité de l'os fémurale, un premier élément de charnière (3) joint à un bout de ladite barre (1) qui est opposé au bout inséré dans le fémur; une seconde barre (2) insérable dans la cavité de l'os tibial; un second élément de charnière (4) joint à un bout de ladite seconde barre (2) qui est opposé au bout inséré dans l'os tibial, un joint articulé entre lesdits deux éléments (3,4) ayant au moins un roulement (5), caractérisée en ce que lesdites barres (1,2) sont jointes à leur respectives éléments de charnière (3,4) au moyen d'un embrèvement à queue d'aronde (10,11; 16,17; 16',17'; 16'',17'').

2. Articulation artificielle selon la revendication 1, caractérisée en ce qu'une entretoise (6,7) est prévu entre ladite barre (1,2) et ledit élément de charnière (3,4).

3. Articulation artificielle selon la revendication 1 ou 2, caractérisée en ce que lesdites entretoises comprendent un embrèvement à queue d'aronde entre ladite barre (1,2) et ledit élément de charnière (3,4).

4. Articulation artificielle selon une des revendications précédentes, caractérisée en ce que au moins un roulement est un roulement autolubrifiant.

5. Articulation artificielle selon les revendications 1 ou 4, caractérisée en ce qu'il sont prévus deux roulement (5).

6. Articulation artificielle selon une des revendications précédentes, caractérisée en ce qu'un moyen de blocage est prévu pour bloquer mécaniquement ledit joint.

7. Articulation artificielle selon la revendication 4, caractérisée en ce que ledit moyen de blocage comprend une cheville (12).

8. Articulation artificielle selon la revendication 7, caractérisée en ce que ladite cheville (12) est une cheville filetée autobloquante.

9. Articulation artificielle selon la revendication 6, caractérisée en ce que ledit moyen bloquant comprend un collet filetée autobloquant.

10. Articulation artificielle selon la revendication 6, caractérisée en ce que ledit moyen bloquant comprend une bague elastique.

11. Articulation artificielle selon une des revendications précédentes, caractérisée en ce que le deux éléments de charnière (3,4) sont formés de façon que l'axe verticale de la premiere barre (1) et l'axe verticale de la seconde barre (2) sont paralleles, mais le deuxième axe est deplacé en avant par rapport au premier axe en direction de la marche du patient.

12. Articulation artificielle selon une des revendications précédentes, caractérisée en ce que des entretoises sont prévues sur lesdites barres (1,2).

13. Articulation artificielle selon une des revendications précédentes, caractérisée en ce que les moyens de blocage comprendent des couvercles (8,9) qui sont assemblés lateralement sur lesdits roulements et sont fixés par des chevilles.
